# FASCICULE DE BREVET EUROPEEN

(11) **EP 2 855 103 B1**
(45) Date de publication et mention de la délivrance du brevet: **12.07.2017**
(21) Numéro de dépôt: 13725369.6
(22) Date de dépôt: 28.05.2013
(51) Int. Cl.: B25J 9/00, A47L 15/42

(54) **BRAS D'EXOSQUELETTE A UN ACTIONNEUR**
EXOSKELETT STELLGLIEDARM
EXOSKELETON ACTUATOR ARM

(30) Priorité: 04.06.2012 FR 1255177
(43) Date de publication de la demande: 08.04.2015
(73) Titulaire: Commissariat à l'Énergie Atomique et aux Énergies Alternatives, 75015 Paris (FR)
(72) Inventeur: GARREC, Philippe, 91190 Gif-sur-Yvette (FR); PERROT, Yann, 91700 Sainte-Geneviève Des Bois (FR); PONSORT, Dominique, 91570 Bièvres (FR); RIGLET, Aurélie, 45230 Dammaries sur Loing (FR)
(74) Mandataire: Parzy, Benjamin Alain
(86) Numéro de dépôt international: PCT/EP2013/060918
(87) Numéro de publication internationale: WO 2013/182452

(56) Documents cités:
- FR-A1- 2 949 669
- US-A- 4 180 870
- JARRASSE N ET AL: "Design and acceptability assessment of a new reversible orthosis", INTELLIGENT ROBOTS AND SYSTEMS, 2008. IROS 2008. IEEE/RSJ INTERNATIONAL CONFERENCE ON, IEEE, PISCATAWAY, NJ, USA, 22 septembre 2008 (2008-09-22), pages 1933-1939, XP031348546, ISBN: 978-1-4244-2057-5
- GARREC P ET AL: "ABLE, an innovative transparent exoskeleton for the upper-limb", INTELLIGENT ROBOTS AND SYSTEMS, 2008. IROS 2008. IEEE/RSJ INTERNATIONAL CONFERENCE ON, IEEE, PISCATAWAY, NJ, USA, 22 septembre 2008 (2008-09-22), pages 1483-1488, XP031348544, ISBN: 978-1-4244-2057-5
- PERRY J C ET AL: "Upper-Limb Powered Exoskeleton Design", IEEE / ASME TRANSACTIONS ON MECHATRONICS, IEEE SERVICE CENTER, PISCATAWAY, NJ, US, vol. 12, no. 4, 1 août 2007 (2007-08-01), pages 408-417, XP011190378, ISSN: 1083-4435, DOI: 10.1109/TMECH.2007.901934

## Description

L'invention est relative à un bras d'exosquelette à un seul actionneur, qui peut avantageusement être fixé à la partie dorsale d'un exosquelette.

### ARRIERE PLAN DE L'INVENTION

On connaît divers bras d'assistance adaptables sur des exosquelettes, utilisables notamment pour soulever des charges par dessous, comme par exemple un sac de sable, ou un malade. En général, ces bras possèdent des articulations d'épaule, de coude et de poignet qui sont associées à des motorisations respectives, ce qui rend l'ensemble lourd et complexe. Voir par exemple le document "Design and acceptability assessment of a new reversible orthosis" publié en 2008 dans la revue INTELLIGENT ROBOTS AND SYSTEMS, qui décrit un bras d'assistance destiné à équiper un exosquelette comprenant une épaule articulée suivant trois axes de rotation et au moins quatre actionneurs. En outre, les motorisations prennent souvent la forme de moteurs transversaux s'étendant dans le prolongement de l'axe de l'articulation, ce qui génère un encombrement important. Pour limiter cet encombrement, il convient dès lors de limiter la charge soulevable, ou d'augmenter le rapport de réduction entre le moteur et le segment entraîné, ce qui tend à diminuer la transparence de l'articulation. Cet inconvénient peut être partiellement gommé par une rétroaction en couple qui nécessite l'utilisation d'un capteur de couple et qui rend la solution moins fiable, plus complexe et plus onéreuse.

D'autres solutions existent, comme des organes de levage adaptables sur un exosquelette qui ne comportent pas d'actionneur, mais un équilibrage à ressort. Un tel organe doit être réglé à l'avance pour une charge donnée, ce qui limite singulièrement son enveloppe d'utilisation.

### OBJET DE L'INVENTION

L'invention a pour objet de proposer un bras d'assistance simplifié pour exosquelette, dont la compacité permet son intégration aisée à un exosquelette ambulatoire, tel que l'exosquelette HERCULE de la société Rb3d.

### RESUME DE L'INVENTION

En vue de la réalisation de ce but, on propose un bras d'assistance destiné à équiper un exosquelette, comportant :
- une platine portant un organe moteur ;
- un segment d'épaule articulé sur la platine au moyen d'une première articulation selon un premier axe sensiblement vertical en service ;
- un segment de bras ayant une extrémité proximale articulée sur le segment d'épaule au moyen d'une deuxième articulation selon un deuxième axe sensiblement perpendiculaire au premier axe ;
- un segment d'avant-bras ayant une extrémité proximale articulée sur une extrémité distale du segment de bras au moyen d'une troisième articulation selon un troisième axe sensiblement parallèle au deuxième axe.

Selon l'invention, le bras d'assistance comporte en outre :
- au moins un câble d'actionnement entraîné par l'organe moteur et formant deux brins qui s'étendent parallèlement au premier axe pour traverser la première articulation et embrasser une poulie de réception montée tournante autour du deuxième axe et qui est solidaire en rotation du bras;
- des moyens de liaison attelés entre le segment d'épaule et le segment d'avant-bras agencés de sorte qu'une flexion du segment de bras entraîne une flexion concomitante du segment de l'avant-bras.

Ainsi, à l'aide d'un unique organe moteur monté sur la platine (donc positionné dans le dos de l'opérateur lorsque la platine est rapportée sur l'exosquelette), il est possible de soulever des charges à l'aide de l'avant-bras. L'ensemble est très compact, et l'organe moteur peut être placé sur la platine, donc dans le dos de l'opérateur, de sorte qu'il ne gêne ni la vision de l'opérateur ni l'amplitude des mouvements du bras d'assistance.

Les directions en service désignent des directions d'articulation alors que l'exosquelette est porté par un opérateur, celui-ci ayant le bras qui coopère avec le bras d'assistance dans une position de repos le long du corps.

Selon un mode de réalisation particulier, l'avant-bras comporte deux sous segments reliés entre eux par une quatrième articulation selon un quatrième axe sensiblement perpendiculaire au troisième axe. Cette disposition permet un mouvement latéral libre de l'extrémité du bras.

### BREVE DESCRIPTION DES DESSINS

L'invention sera mieux comprise à la lumière de la description détaillée qui suit d'un mode particulier de réalisation en référence aux figures des dessins annexés parmi lesquelles :

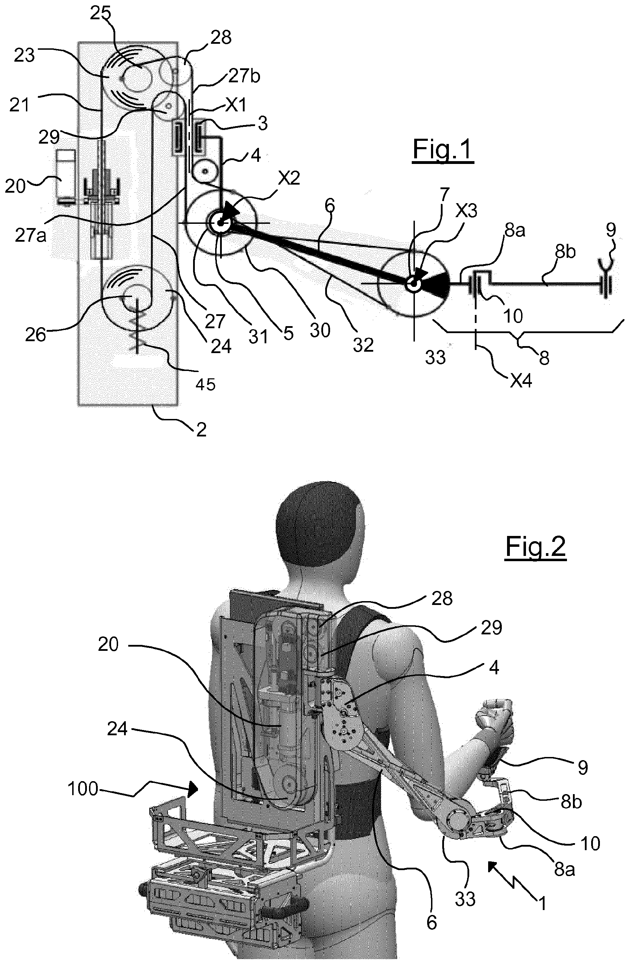

- la figure 1 est un schéma de principe du bras d'assistance de l'invention ;
- la figure 2 est une vue d'un exosquelette équipé du bras d'assistance de l'invention et porté par un utilisateur.

### DESCRIPTION DETAILLEE DE L'INVENTION

En référence aux figures, le bras d'assistance 1 de l'invention comporte tout d'abord une platine 2 qui porte une première articulation 3 permettant d'articuler un segment d'épaule 4 selon un premier axe X1 vertical en service. La platine 2 est avantageusement équipée de moyens de sa solidarisation à la partie dorsale d'un exosquelette 100, ou à tout le moins une structure support portée par un opérateur.

Le segment d'épaule 4 porte une deuxième articulation 5 permettant d'articuler l'extrémité proximale d'un segment de bras 6 selon un deuxième axe X2 (ici vu en bout) sensiblement perpendiculaire au premier axe X1. L'extrémité distale du segment de bras 6 porte une troisième articulation 7 qui permet d'articuler l'extrémité proximale d'un segment d'avant-bras 8 selon un troisième axe X3 (ici vu en bout) sensiblement parallèle au deuxième axe X2. Le segment d'avant-bras 8 a une extrémité distale qui porte un repose-main 9. Ici, le segment d'avant-bras 8 est décomposé en deux sous-segments 8a, 8b reliés entre eux par une quatrième articulation 10 permettant d'articuler entre eux les deux sous-segments selon un quatrième axe X4 sensiblement perpendiculaire au troisième axe X3.

Le bras d'assistance 1 de l'invention est pourvu d'un organe moteur 20 porté par la platine 2, ici un moteur électrique, apte à entraîner un premier câble 21 ou câble d'entraînement par l'intermédiaire d'un vérin à câble 22. Le câble d'entraînement 21 comporte un brin tendu entre deux premières poulies 23, 24 montées tournantes sur la platine 2. La première poulie 24 est ici munie d'un tendeur 45 pour maintenir une tension dans le câble d'entraînement 21. Les premières poulies 23,24 sont solidaires en rotation respectivement de deuxièmes poulies 25,26 de diamètre inférieur à celui des premières poulies 23,24. Sur les deuxième poulies 25,26 est attelé un deuxième câble 27 ou câble d'actionnement qui, grâce à deux poulies de renvoi 28,29 montées tournantes sur la platine 2, forme deux brins 27a,27b qui s'étendent parallèlement au premier axe X1 en passant à l'intérieur de la première articulation 3, qui est creuse. Puis les deux brins parallèles 27a, 27b se séparent pour embrasser une poulie de réception 30 montée tournante autour du deuxième axe X2 en étant solidaire en rotation du segment de bras 6.

Ainsi, lorsque le moteur électrique 20 est commandé, il provoque le déplacement du câble d'entraînement 21 dans un sens ou dans l'autre, provoquant alors un déplacement du câble d'actionnement 27, ce qui provoque une flexion du segment de bras 6. Le passage des brins 27a,27b du deuxième câble dans la première articulation (qui est libre) permet une libre rotation du segment d'épaule sans provoquer de flexion du segment de bras 6. L'utilisation de deux câbles enroulés autour de poulies de diamètres distincts permet une démultiplication de couple.

Le bras de l'invention est par ailleurs équipé de moyens de liaison permettant de générer une flexion du segment d'avant-bras 8 en réponse à une flexion du segment de bras 6. En l'occurrence ici, ces moyens comprennent une poulie de coordination 31 montée fixe sur le segment d'épaule 4 concentriquement au deuxième axe X2. Un troisième câble ou câble de coordination 32 est attelé à la poulie de coordination 31 et embrasse après croisement des brins une poulie menante 33 montée tournante autour du troisième axe X3 en étant solidaire en rotation du segment d'avant-bras 8. Grâce à ces moyens de liaison, une flexion du segment de bras 6 commandée par le moteur 20 provoque une flexion concomitante et de même sens du segment d'avant-bras 8. En faisant varier le diamètre respectif de la poulie de coordination et de la poulie menante, on règle précisément l'amplitude de la flexion du segment d'avant-bras en fonction de l'amplitude de la flexion du segment de bras.

Un tel bras d'assistance présente plusieurs avantages. Il ne fait tout d'abord appel qu'à un seul organe moteur, logé dans le dos de l'opérateur. Ensuite, il permet le levage d'une charge par-dessous. Par ailleurs, l'actionnement par vérin à câble permet le positionnement d'un moteur long monté à plat sur la platine (et donc dans le dos de l'utilisateur) conduisant à un bras d'assistance très mince et une transmission très transparente (faibles inertie et frottement), permettant de se passer d'une rétroaction par capteur de couple. Enfin, le bras est autonome et peut être facilement démonté de l'exosquelette.

L'invention n'est pas limitée à ce qui vient d'être décrit, mais englobe au contraire toute variante entrant dans le cadre défini par les revendications.

En particulier, bien que l'organe de liaison entre le segment d'épaule et le segment d'avant bras comprenne ici deux poulies et un câble de coordination associé, on pourra utiliser d'autres organes de liaison comme une bielle attelée entre le segment d'épaule et le segment d'avant-bras.

Bien que l'actionnement fasse ici appel à un vérin à câble à boucle double avec deux câbles permettant d'augmenter l'effort du vérin avec une perte d'énergie négligeable, on pourra utiliser un actionnement à câble unique. Dans ce cas, le vérin à câble agira directement sur le câble d'actionnement.

## Revendications

1. Bras d'assistance destiné à équiper un exosquelette, comportant :
- une platine (2) portant un organe moteur (20) ;
- un segment d'épaule (4) articulé sur la platine au moyen d'une première articulation (3) selon un premier axe (X1) sensiblement vertical en service ;
- un segment de bras (6) ayant une extrémité proximale articulée sur le segment d'épaule au moyen d'une deuxième articulation (5) selon un deuxième axe (X2) sensiblement perpendiculaire au premier axe ;
- un segment d'avant-bras (8) ayant une extrémité proximale articulée sur une extrémité distale du segment de bras au moyen d'une troisième articulation (7) selon un troisième axe (X3) sensiblement parallèle au deuxième axe ;
le bras d'assistance étant **caractérisé en ce qu'**il comporte en outre :
- au moins un câble d'actionnement (27) entraîné par l'organe moteur et formant deux brins (27a,27b) qui s'étendent parallèlement au premier axe pour traverser la première articulation et s'enrouler autour d'une poulie de réception (30) montée tournante autour du deuxième axe et qui est solidaire en rotation du bras ;
- des moyens de liaison (31,32,33) attelés entre le segment d'épaule et le segment d'avant-bras agencés de sorte qu'une flexion du segment de bras entraîne une flexion concomitante du segment d'avant-bras.

2. Bras d'assistance selon la revendication 1, dans lequel l'organe moteur (20) est associé à un vérin à câble (22) qui entraine un câble d'entraînement (21) tendu entre deux premières poulies (23,24) montées tournantes sur la platine (2), le câble d'actionnement (27) étant quant à lui attelé à deux deuxièmes poulies (25,26) respectivement solidaires des premières poulies (23,24) et ayant un diamètre inférieur à un diamètre des premières poulies.

3. Bras d'assistance selon la revendication 1, dans lequel le segment d'avant-bras comporte une extrémité distale pourvue d'un repose-main (9).

4. Bras d'assistance selon la revendication 1, dans lequel les moyens de liaison entre le segment d'épaule (4) et le segment d'avant-bras (8) comprend une poulie de coordination (31) concentrique au deuxième axe (X2) mais fixe sur le segment d'épaule, une poulie menante (33) montée tournante autour du troisième axe (X3) en étant solidaire du segment d'avant-bras, et un câble de coordination (32) enroulé avec croisement des brins sur la poulie de coordination et la poulie menante.

5. Bras d'assistance selon la revendication 1, dans lequel le segment d'avant-bras (8) comprend deux sous segments (8a,8b) articulé entre eux au moyen d'une quatrième articulation selon un quatrième axe (X4) sensiblement perpendiculaire au troisième axe (X3).

## Patentansprüche

1. Hilfsarm, der dazu bestimmt ist, an einem Exoskelett angebracht zu werden, umfassend:
- eine Platte (2), die ein Antriebsorgan (20) trägt;
- ein Schultersegment (4), das an der Platte mittels einer ersten Gelenkverbindung (3) um eine erste Achse (X1) angelenkt ist, die im Gebrauch im Wesentlichen vertikal ist;
- ein Armsegment (6), das ein proximales Ende hat, das an dem Schultersegment mittels einer zweiten Gelenkverbindung (5) um eine zweite Achse (X2) angelenkt ist, die im Wesentlichen senkrecht zur ersten Achse ist;
- ein Unterarmsegment (8), das ein proximales Ende hat, das an einem distalen Ende des Armsegments mittels einer dritten Gelenkverbindung (7) um eine dritte Achse (X3) angelenkt ist, die im Wesentlichen parallel zur zweiten Achse ist;
wobei der Hilfsarm **dadurch gekennzeichnet ist, dass** er ferner umfasst:
- mindestens ein Betätigungsseil (27), das von dem Antriebsorgan angetrieben wird und zwei Stränge (27a, 27b) bildet, die sich parallel zur ersten Achse erstrecken, um durch die erste Gelenkverbindung hindurchzugehen und sich um eine Aufnahmescheibe (30) zu wickeln, die drehbar um die zweite Achse gelagert und drehfest mit dem Arm verbunden ist;
- Verbindungsmittel (31, 32, 33), die zwischen dem Schultersegment und dem Unterarmsegment gekoppelt sind, welche so ausgebildet sind, dass eine Beugung des Armsegments eine gleichzeitige Beugung des Unterarmsegments verursacht.

2. Hilfsarm nach Anspruch 1, bei dem das Antriebsorgan (20) mit einer Seilwinde (22) verbunden ist, die ein Antriebsseil (21) antreibt, das zwischen zwei ersten Scheiben (23, 24) gespannt ist, die drehbar an der Platte (2) gelagert sind, wobei das Betätigungsseil (27) selbst an zwei zweite Scheiben (25, 26) gekoppelt ist, die jeweils fest mit den ersten Scheiben (23, 24) verbunden sind und einen Durchmesser haben, der kleiner als ein Durchmesser der ersten Scheiben ist.

3. Hilfsarm nach Anspruch 1, bei dem das Unterarmsegment ein distales Ende umfasst, das mit einer Handstütze (9) versehen ist.

4. Hilfsarm nach Anspruch 1, bei dem die Verbindungsmittel zwischen dem Schultersegment (4) und dem Unterarmsegment (8) eine Koordinationsscheibe (31) umfassen, die konzentrisch zur zweiten Achse (X2), jedoch an dem Schultersegment fixiert ist, eine Treibscheibe (33), die drehbar um die dritte Achse (X3) gelagert ist, während sie fest mit dem Unterarmsegment verbunden ist, sowie ein Koordinationsseil (32), das mit einer Kreuzung der Stränge um die Koordinationsscheibe und die Treibscheibe gewickelt ist.

5. Hilfsarm nach Anspruch 1, bei dem das Unterarmsegment (8) zwei Untersegmente (8a, 8b) umfasst, die aneinander mittels einer vierten Gelenkverbindung um eine vierte Achse (X4) angelenkt sind, die im Wesentlichen senkrecht zur dritten Achse (X3) ist.

## Claims

1. An assist arm intended to be fitted to an exoskeleton, comprising:
- a mounting plate (2) carrying a motor component (20);
- a shoulder segment (4) articulated on the mounting plate by means of a first articulation (3) about a first axis (X1) which is substantially vertical during use;
- an arm segment (6) having a proximal end articulated on the shoulder segment by means of a second articulation (5) about a second axis (X2) which is substantially perpendicular to the first axis;
- a forearm segment (8) having a proximal end articulated on a distal end of the arm segment by means of a third articulation (7) about a third axis (X3) which is substantially parallel to the second axis;
the assist arm being **characterized in that** it furthermore comprises:
- at least one actuation cable (27) driven by the motor component and forming two strands (27a, 27b), which extend parallel to the first axis so as to pass through the first articulation and be wound around a receiving pulley (30) which is mounted so as to rotate about the second axis and is linked in rotation with the arm;
- connecting means (31, 32, 33) which are attached between the shoulder segment and the forearm segment and are arranged so that flexion of the arm segment leads to concomitant flexion of the forearm segment.

2. The assist arm as claimed in claim 1, wherein the motor component (20) is associated with a cable actuator (22) which drives a drive cable (23) tensioned between two first pulleys (23, 24) mounted so as to rotate on the mounting plate (2), the actuation cable (27) being for its part attached to two second pulleys (25, 26) respectively linked to the first pulleys (23, 24) and having a diameter less than a diameter of the first pulleys.

3. The assist arm as claimed in claim 1, wherein the forearm segment comprises a distal end provided with a handrest (9).

4. The assist arm as claimed in claim 1, wherein the connecting means between the shoulder segment (4) and the forearm segment (8) comprise a coordination pulley (31) concentric with the second axis (X2) but fixed on the shoulder segment, a drive pulley (33) mounted so as to rotate about the third axis (X3) while being linked to the forearm segment, and a coordination cable (32) wound with strand crossover on the coordination pulley and the drive pulley.

5. The assist arm as claimed in claim 1, wherein the forearm segment (8) comprises two subsegments (8a, 8b) articulated to one another by means of a fourth articulation about a fourth axis (X4) which is substantially perpendicular to the third axis (X3).
